# EUROPEAN PATENT SPECIFICATION

(11) **EP 3 941 288 B1**
(45) Date of publication and mention of the grant of the patent: **29.04.2026**
(21) Application number: 20721422.2
(22) Date of filing: 17.03.2020
(51) Int. Cl.: A24F 40/44, A61M 11/04, A61M 15/06

(54) **AEROSOL DELIVERY SYSTEM**
AEROSOLABGABESYSTEM
SYSTÈME D'ADMINISTRATION D'AÉROSOL

(30) Priority: 21.03.2019 EP 19164454
(43) Date of publication of application: 26.01.2022
(73) Proprietor: Imperial Tobacco Limited, Bristol, BS3 2LL (GB)
(72) Inventor: LORD, Chris, Liverpool Merseyside L24 9HP (GB); SUDLOW, Thomas, Liverpool Merseyside L24 9HP (GB); ILLIDGE, Ben, Liverpool Merseyside L24 9HP (GB); MADDEN, Alfred, Liverpool Merseyside L24 9HP (GB); ASTBURY, Ben, Liverpool Merseyside L24 9HP (GB)
(74) Representative: Mewburn Ellis LLP
(86) International application number: PCT/EP2020/057303
(87) International publication number: WO 2020/187916

(56) References cited:
- WO-A1-2015/117704
- GB-A- 2 561 958
- US-A1- 2007 102 013
- US-A1- 2015 027 459
- US-A1- 2018 140 018

## Description

### Field of the Invention

The present invention relates to an aerosol delivery system and an aerosol-generation apparatus for an aerosol delivery system. In particular, the present invention relates to an aerosol delivery system comprising a heater configured to heat an aerosol precursor to generate an aerosolised composition for inhalation by a user, and to an aerosol-generation apparatus therefor.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette^{®} Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, *inter alia,* as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat. GB2561958 discloses an aerosol-generation apparatus comprises a heater having heating elements at the heating surface, and wherein the heating surface comprises a fluid transport region adjacent the heating element(s). The fluid transport region moves aerosol precursor liquid across the heating surface towards the heating element(s).

The present invention has been devised in light of the above considerations.

### Summary of the Invention

An invention is set out in the claims. At its most general, the present invention proposes that dried conductive fluid is used to form at least one heater element on an activation surface of a fluid-transfer article. The activation surface has at least one channel which opens outward. The fluid-transfer article may then act as a reservoir for holding an aerosol precursor, and for transferring that aerosol precursor to the activation surface. The aerosol precursor can then be heated by the heater element or elements to form vapour or a vapour/aerosol mixture which can then pass to a user.

The heater element or elements are preferably formed on parts of the activation surface other than the or each channel. The element or elements may prevent or restrict aerosol precursor leaving the fluid-transfer article from regions where they are formed, and so the or each channel provides a region where the aerosol precursor may leave the fluid-transfer article (e.g. as vapour or a mixture of vapour and aerosol) in an unrestricted way. The channel, or the channels together, may thus form an air-flow pathway along the activation surface. The heater elements may extend on to the side walls of the or each channel, to increase the heat transfer.

Thus, there may be provided an aerosol-generation apparatus comprising a heater and a fluid-transfer article, the fluid-transfer article having a first region for holding an aerosol precursor and for transferring said aerosol precursor to an activation surface of a second region of said article, wherein said second region comprises at least one discontinuity in said activation surface to form a corresponding at least one channel in said activation surface, the or each said channel being configured for providing an air-flow pathway across said activation surface and opening in a direction away from said first region, said heater having at least one heater element formed on said activation surface, said at least one heater element being of a dried conductive fluid with electrical connections thereto.

Optionally, said activation surface is disposed at an end of said article.

Preferably, wherein said at least one heater element is formed on parts of said activation surface other than the or each discontinuity, which forms the or each channel.

Normally, at least parts of said at least one heating element are formed on parts of said activation surface between said channels.

Advantageously, the or each said channel may be at least partly defined by a pair of spaced apart side walls, and an arcuate surface portion extending between said wall portions to form a ceiling portion of said channel.

Optionally, said arcuate surface portion blends smoothly with each of said side walls, thereby eliminating a sharp corner therebetween.

Alternatively, the or each channel may be at least partially defined by a pair of spaced apart side walls and a flat surface portion, said flat surface portion extending between said wall portions to form a ceiling portion of said channel. A further possibility is that the or each channel is at least partially defined by a pair of side walls, said side walls being inclined relative to each other to meet an apex portion of said channel.

In such arrangements, the at least one heater element may be formed on at least parts of said side walls, but preferably not on any ceiling portion.

Conveniently, said side walls are substantially planar.

Conveniently, at least said second region is formed from a polymeric wicking material.

Advantageously, said first and second regions are both formed from said polymeric wicking material.

Optionally, said polymeric wicking material is porous.

Conveniently, said polymeric wicking material is configured such that pore diameter in said first region is greater than pore diameter in said second region.

Advantageously, said polymeric wicking material is heat resistant.

Optionally, said polymeric wicking material is a hydrophilic material that is configured to transfer fluid from said first region to said second region.

Conveniently, said polymeric wicking material is of greater hydrophilicity in said second region than said first region.

According to a second aspect of the present invention, there may be provided an aerosol-delivery system comprising an aerosol-generation apparatus as discussed above, and a carrier, the carrier having a housing containing the heater and the fluid-transfer article. In such an aerosol-delivery system, the housing may have an inlet and outlet, with the air-flow pathway extending to the inlet and outlet.

According to a third aspect of the present invention, there may be provided a method of forming an aerosol-generation device comprising: forming a fluid-transfer article, the fluid-transfer article having a first region for holding an aerosol precursor and for transferring said aerosol precursor to an activation surface of a second region of said article, wherein said second region comprises at least one discontinuity in said activation surface to form a corresponding at least one channel in said activation surface, the or each said channel being configured for providing an air-flow pathway across said activation surface and opening in a direction away from said first region; dipping said activation surface in a conductive fluid to coat at least a part of said activation surface with said conductive fluid; drying said conductive fluid to form heater elements; and making electrical connection to said dried conductive fluid, thereby to form a heater on said fluid-transfer article.

The invention includes the combination of the aspects and preferred features described except where such a combination is clearly impermissible or expressly avoided.

### Summary of the Figures

So that the invention may be more readily understood, and so that further features thereof may be appreciated, embodiments of the invention will now be described by way of example with reference to the accompanying drawings in which:
**Figure 1** is a perspective view illustration of a system for aerosol delivery according to one or more embodiments of the present invention;
**Figure 2** is a cross-sectional side view illustration of part of an apparatus of the system for aerosol delivery of Figure 1;
**Figure 3** is a cross-sectional side view illustration of the system and apparatus for aerosol delivery of Figure 1;
**Figure 4** is a perspective view illustration of an aerosol carrier for use in the system for aerosol delivery according to one or more embodiments of the present invention;
**Figure 5** is a cross-section side view of elements of an aerosol carrier and of part of an apparatus of the system for aerosol delivery according to one or more embodiments of the present invention;
**Figure 6** is a cross-section side view of elements of an aerosol carrier and of part of an apparatus of the system for aerosol delivery according to one or more embodiments of the present invention;
**Figure 7** is a perspective view illustration of the aerosol carrier and of part of an apparatus of the system for aerosol delivery according to one or more embodiments of the present invention;
**Figure 8** is a perspective view illustration of the aerosol carrier and of part of an apparatus of the system for aerosol delivery according to one or more embodiments of the present invention;
**Figure 9** is a perspective end view illustration of a fluid-transfer article of the aerosol carrier according to one or more embodiments of the present invention;
**Figure 10** is a perspective end view illustration of a fluid-transfer article of the aerosol carried according to one or more embodiments of the present invention;
**Figure 11** is a cross-section side view of an aerosol carrier according to one or more embodiments of the present invention;
**Figure 12** is a perspective cross-section side view of the aerosol carrier of Figure 11;
**Figure 13** is an exploded perspective view illustration of a kit-of-parts for assembling a system according to one or more embodiments of the present invention;
**Figure 14** is a cross-section side view of elements of an aerosol carrier and of part of an apparatus of the system for aerosol delivery according to one or more embodiments of the present invention;
**Figure 15** is a cross-section view of elements of an aerosol carrier and of part of an apparatus of the system for aerosol delivery according to one or more embodiments of the present invention; and
**Figure 16** is a perspective view of a fluid-transfer article of the system for aerosol delivery according to one or more embodiments of the present invention;

### Detailed Description of the Invention

Aspects and embodiments of the present invention will now be discussed with reference to the accompanying figures.

In general outline, one or more embodiments in accordance with the present invention may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

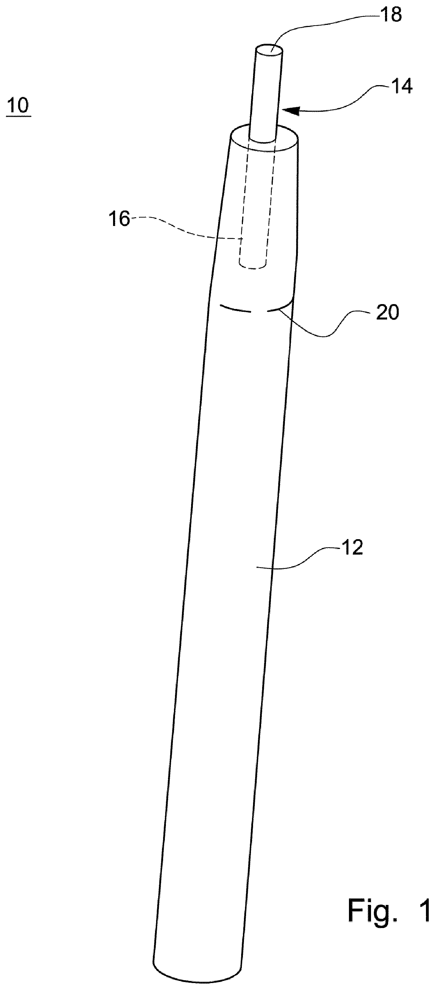

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end 16 thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article to the second end 18.

The device 12 also comprises air-intake apertures 20 in the housing of the apparatus 12 to provide a passage for air to be drawn into the interior of the apparatus 12 (when the user sucks or inhales) for delivery to the first end 16 of the aerosol carrier 14, so that the air can be drawn across an activation surface of a fluid-transfer article located within a housing of the aerosol carrier cartridge 14 during use. Optionally, these apertures may be perforations in the housing of the apparatus 12.

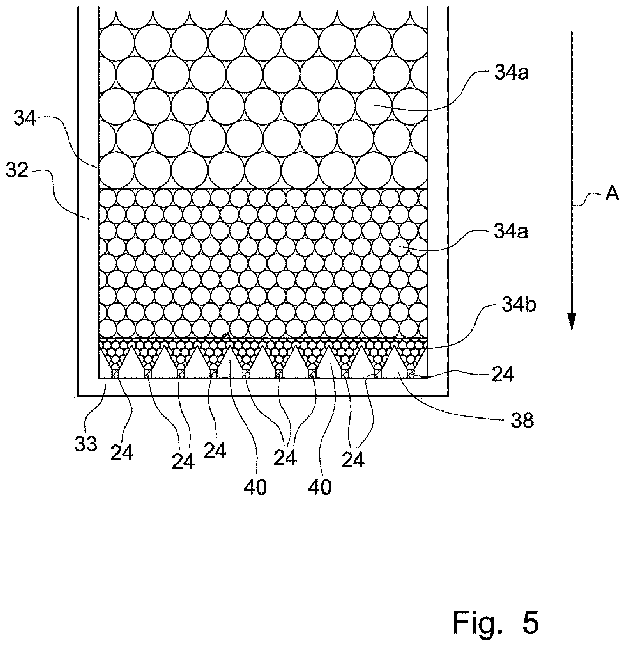

A fluid-transfer article (not shown in Figure 1, but described hereinafter with reference to Figs. 5, 6, 7, 8, 9, 10, 11 and 12) is located within a housing of the aerosol carrier 14. The fluid-transfer article contains an aerosol precursor material, which may include at least one of: nicotine; a nicotine precursor material; a nicotine compound; and one or more flavourings. The fluid-transfer article is located within the housing of the aerosol carrier 14 to allow air drawn into the aerosol carrier 14 at, or proximal, the first end 16 to flow across an activation surface of the fluid-transfer article. As air passes across the activation surface of the fluid-transfer article, an aerosol may be entrained in the air stream from a substrate forming the fluid-transfer article, e.g. via diffusion from the substrate to the air stream and/or via vaporisation of the aerosol precursor material and release from the fluid-transfer article under heating.

The substrate forming the fluid-transfer article 34 comprises a porous material where pores of the porous material hold, contain, carry, or bear the aerosol precursor material. In particular, the porous material of the fluid-transfer article may be a polymeric wicking material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex^{®}. Further optionally, a substrate forming the fluid-transfer article may comprise Polypropylene (PP) or Polyethylene Terephthalate (PET). All such materials may be described as heat resistant polymeric wicking material in the context of the present invention.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

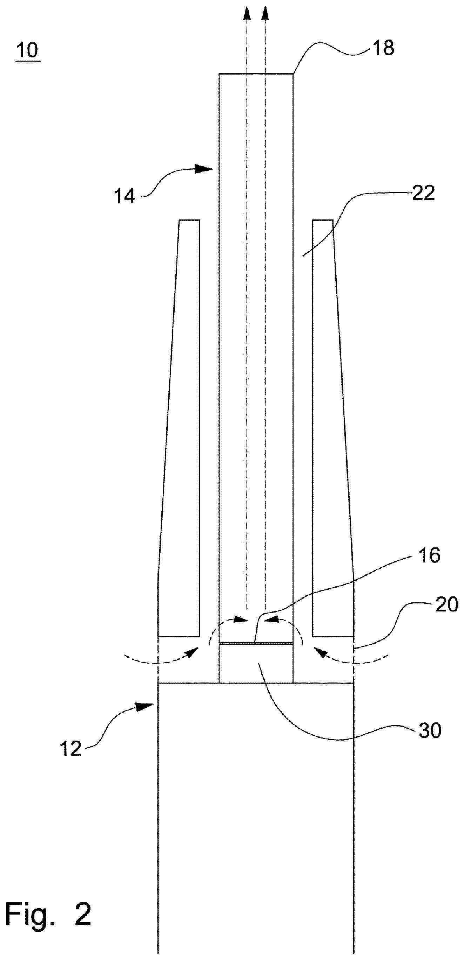

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprise a heater which will be described in more detail later.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows into the aerosol carrier 14, it passes across the activation surface of the fluid-transfer article. Heat from the heater causes vaporisation of aerosol precursor material at the activation surface of the fluid-transfer article and an aerosol is created in the air flowing over the activation surface. Thus, through the application of heat in the region of the activation surface of the fluid-transfer article, an aerosol is released, or liberated, from the fluid-transfer article, and is drawn from the material of the aerosol carrier unit by the air flowing across the activation surface and is transported in the air flow to via outlet conduits (not shown in Figure 2) in the housing of the aerosol carrier 14 to the second end 18. The direction of air flow is illustrated by arrows in Figure 2.

To achieve release of the captive aerosol from the fluid-transfer article, the fluid-transfer article of the aerosol carrier 14 is heated by the heater. As a user sucks or inhales on second end 18 of the aerosol carrier 14, the aerosol released from the fluid-transfer article and entrained in the air flowing across the activation surface of the fluid-transfer article is drawn through the outlet conduits (not shown) in the housing of the aerosol carrier 14 towards the second end 18 and onwards into the user's mouth.

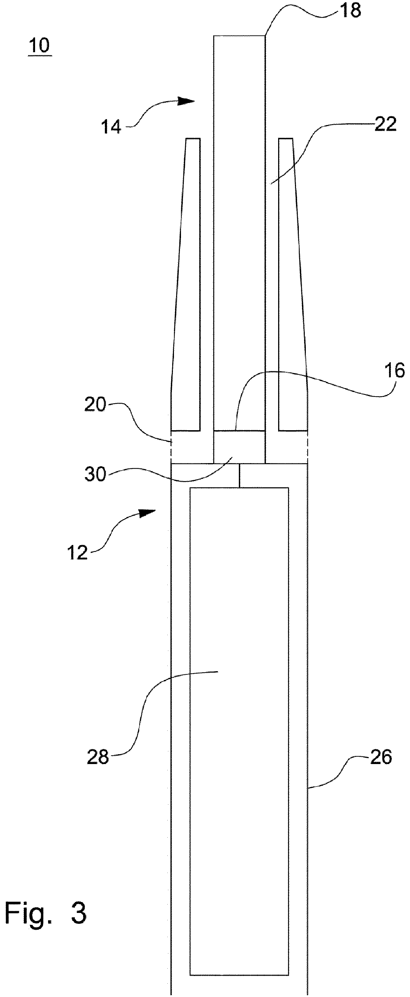

Turning now to Figure 3, a cross-sectional side view of the aerosol delivery system 10 is schematically illustrated showing the features described above in relation to Figures 1 and 2 in more detail. As can be seen, apparatus 12 comprises a housing 26, in which are located the receptacle 22 and heater. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater.

Responsive to activation of the control circuitry of apparatus 12, the heater heats the fluid-transfer article (not shown in Figure 3) of aerosol carrier 14. This heating process initiates (and, through continued operation, maintains) release of vapour and/or an aerosol from the activation surface of the fluid-transfer article. The vapour and/or aerosol formed as a result of the heating process is entrained into a stream of air being drawn across the activation surface of the fluid-transfer article (as the user sucks or inhales). The stream of air with the entrained vapour and/or aerosol passes through the aerosol carrier 14 via outlet conduits (not shown) and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

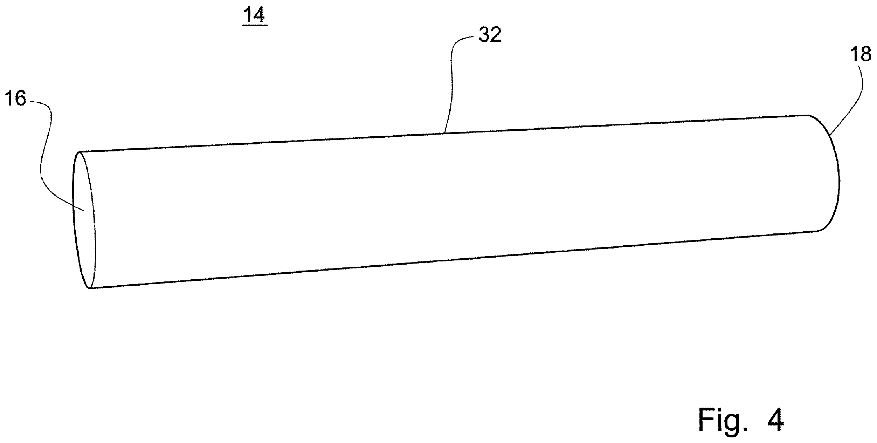

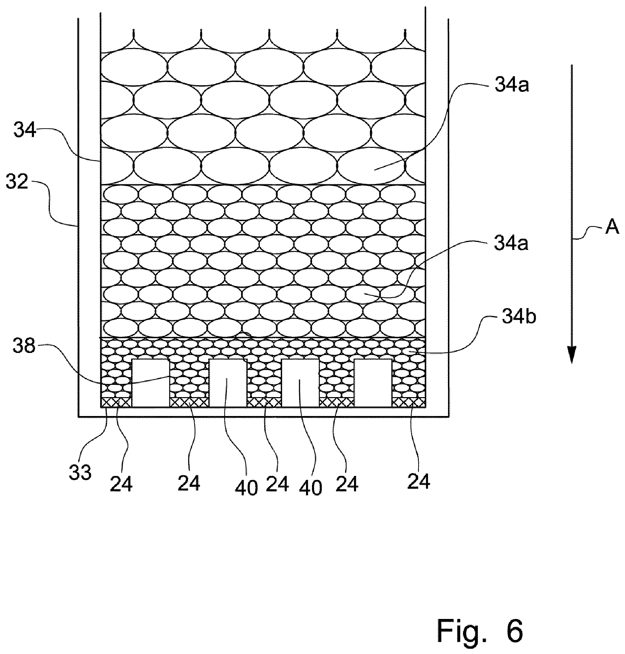

Figures 4 to 6 schematically illustrate the aerosol carrier 14 in more detail (and, in Figures 5 and 6, features within the receptacle in more detail). Figure 4 illustrates an exterior of the aerosol carrier 14, Figure 5 illustrates internal components of the aerosol carrier 14 in an optional arrangement, and Fig. 6 illustrates internal components of the aerosol carrier 14 in another optional arrangement.

Fig. 4 illustrates the exterior of the aerosol carrier 14, which comprises housing 32 for housing said fluid-transfer article (not shown) and at least one other internal component. The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Figure 5 illustrates some internal components of the aerosol carrier 14 and of the heater 24 of apparatus 12.

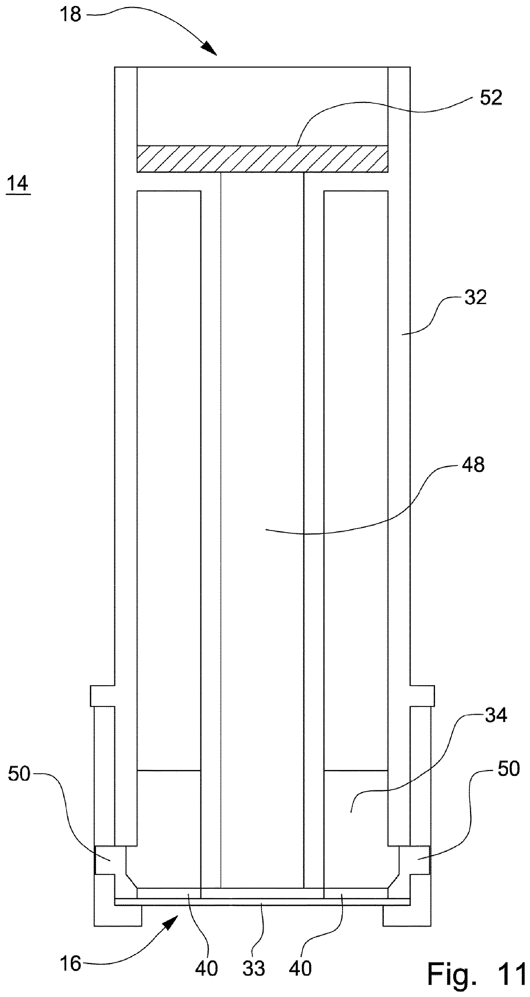

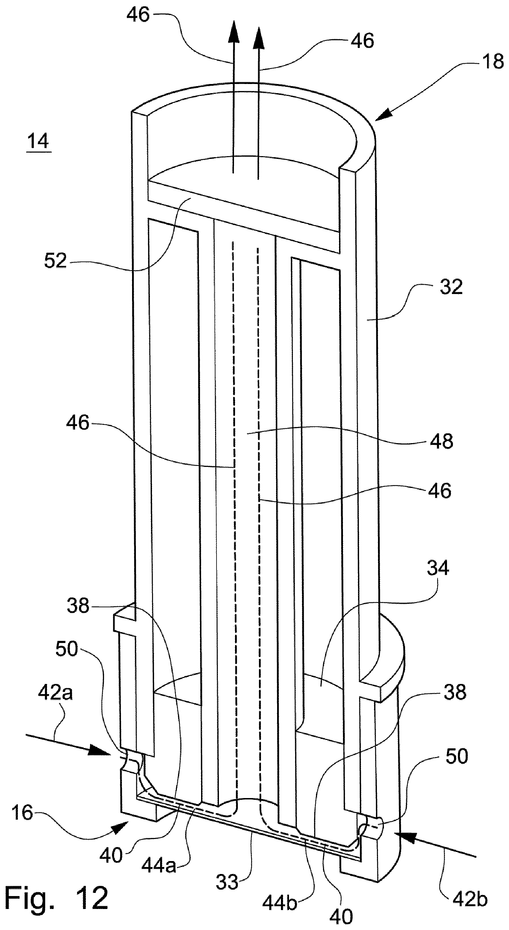

Further components not shown in Figures. 5 and 6 (see Figures 11 and 12) comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material and for providing the aerosol precursor material to the fluid-transfer article 34.

In Figures 5 and 6, the aerosol carrier is shown as comprising the fluid-transfer article 34 located within housing 32. The material forming the fluid transfer article 34 comprises a porous structure, where pore diameter size varies between one end of the fluid-transfer article 34 and another end of the fluid-transfer article. In the illustrative examples of Figs. 5 and 6, the pore diameter size gradually decreases from a first end remote from heater 24 (the upper end as shown in the figure) to a second end proximal heater 24 (the lower end as shown in the figure). Although the figure illustrates the pore diameter size changing in a step-wise manner from the first to the second end (i.e. a first region with pores having a diameter of a first size, a second region with pores having a diameter of a second, smaller size, and a third region with pores having a diameter of a third, yet smaller size), the change in pore size from the first end to the second end may be gradual rather than step-wise. This configuration of pores having a decreasing diameter size from the first end and second end can provide a wicking effect, which can serve to draw fluid from the first end to the second end of the fluid-transfer article 34.

The fluid-transfer article 34 comprises a first region 34a for holding an aerosol precursor. In one or more arrangements, the first region 34a of the fluid-transfer article 34 comprises a reservoir for holding the aerosol precursor. The first region 34a can be the sole reservoir of the aerosol carrier 14, or it can be arranged in fluid communication with a separate reservoir, where aerosol precursor is stored for supply to the first region 34a.

The fluid-transfer article 34 also comprises a second region 34b. Aerosol precursor is drawn from the first region 34a to the second region 34b by the wicking effect of the substrate material forming the fluid transfer article. Thus, the first region 34a is configured to transfer the aerosol precursor to the second region 34b of the article 34.

At the second end of fluid-transfer article 34, the surface of the second region 34b defines an activation surface 38. The activation surface 38 is discontinuous such that at least one channel 40 is formed in the activation surface 38. In some arrangements, the discontinuities may be such that the activation surface 38 is undulating.

In the illustrative examples of Figures 5 and 6, the activation surface 38 comprises a plurality of grooves or valleys therein to form an undulating surface, the grooves or valleys being disposed in a parallel arrangement across the activation surface 38. Thus, there are a plurality of channels 40 in the activation surface 38.

In the illustrative example of Figure 5, the grooves or valleys in the activation surface 38 provide alternating peaks and troughs that give rise to a "saw-tooth" type profile. In one or more optional arrangements, the activation surface may comprise a "castellated" type profile (i.e. a "square wave" type profile), for example, such as illustrated in the example of Figure. 6. In one or more optional arrangements, the activation surface may comprise a "sinusoidal" type profile. The profile may comprise a mixture of two or more of the above profiles given as illustrative examples.

In the illustrative examples of Figures 5 and 6, the first region 34a of the fluid-transfer article 34 is located at an "upstream" end of the fluid-transfer article 34 and the second region 34b is located at a downstream" end of the fluid-transfer article 34. That is, aerosol precursor is wicked, or is drawn, from the "upstream" end of the fluid-transfer article 34 to the "downstream" end of the fluid-transfer article 34 (as denoted by arrow A in Figure 5).

The aerosol precursor is configured to release an aerosol and/or vapour upon heating. Thus, when the activation surface 38 receives heat conveyed from the heater, the aerosol precursor held at the activation surface 38 is heated. The aerosol precursor, which is captively held in material of the fluid-transfer article at the activation surface 38 is released into an air stream flowing through the channels 40.

The shape and/or configuration of the activation surface 38 and the associated shape(s) and/or configuration(s) of the one or more channels 40 formed in the activation surface permit air to flow across the activation surface 38 (through the one or more channels 40) and also increase the surface area of the activation surface 38 of the fluid-transfer article 34 that is available for contact with a flow of air across the activation surface 38.

As mentioned above, the apparatus has a heater. In the illustrated examples of Figures 5 and 6, the heater is formed by conductive fluid which is applied to parts of the activation surface 38 and dried thereon, to form conductive heater elements 24. In Figure 5, the conductive elements 24 are formed on the peaks of the activation surface 38, and in Figure 6 they are formed on the lowermost part of the castellations. The heater elements 24 are connected e.g. to the battery 28 via e.g. suitable electrical connections of the coupling 30.

In accordance with one preferred arrangement, the heater elements 24 are formed by dipping the activation surface 38 into liquid conductive fluid, so that the conductive fluid adheres or otherwise attaches to the appropriate parts of the activation surface 38. The conductive material is then dried, so that the conductive fluid becomes solid, thereby forming the heater elements 24. A material generally known as carbo e-therm may be used as the conductive fluid, although other known conductive fluids may be used instead. Dipping of the activation surface 38 in to the conductive fluid thus becomes a simple way to produce the heater in contact with the activation surface 38, the heater elements 24 forming the active part of that heater.

In the arrangement of Figure 5, the heater elements 24 are formed only on the peaks of the activation surface, with the rest of the activation surface 38 being exposed in the channels 40. The heater elements 24 may limit or restrict the release of the aerosol and /or vapour on heating, as they cover parts of the activation surface, but the size of the heater elements 24 will also affect the amount of heat that can be transferred to the fluid-transfer article 34. Hence, it may be necessary to make the heater elements larger, so that they extend at least partially on the side walls of the channels 40 between the peaks and troughs in Figure 5. Similarly, in the arrangement of Figure 6, the heater elements 24 are shown on the flat surfaces of the castellations, but they may again extend on the side walls thereof if a greater heating area is needed.

In the illustrative examples of Figures 5 and 6, the heater elements 24 are also in contact with a base plate 33 of the casing 32, which base plate 33 is between the channels 40 and the coupling 30. It is possible, however, for there to be a gap between the heater elements 24 and the base plate 33. In such a case, air may pass from one channel 40 to another around the heater elements 24.

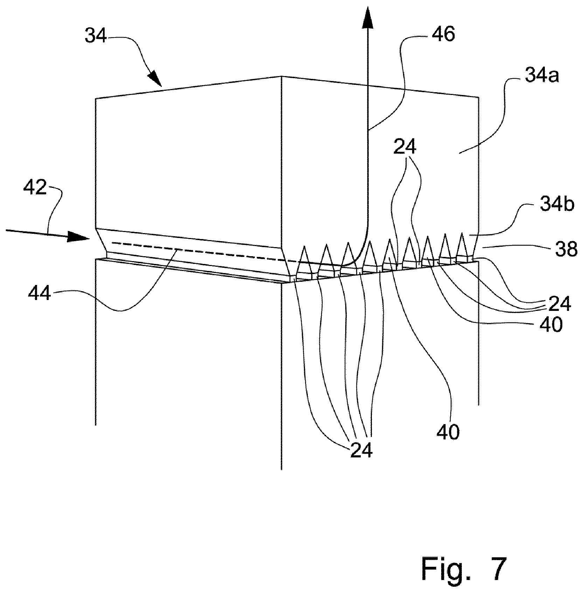

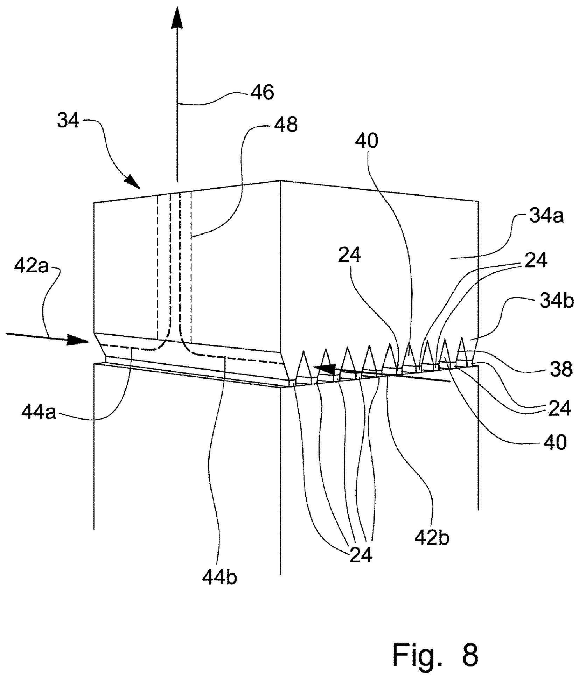

Figures 7 and 8 show perspective view illustrations of the fluid-transfer article 34 of aerosol carrier and heater elements 24 of the apparatus of the system for aerosol delivery. In particular, these figures illustrate air flows across the activation surface 38 when the apparatus is in use in a first arrangement of the fluid-transfer article 34 (see Figure 7), and in a second arrangement of the fluid-transfer article 34 (see Figure 8).

In the illustrated example of use of the apparatus schematically illustrated in Figure 7, when a user sucks on a mouthpiece of the apparatus, air is drawn into the carrier through inlet apertures (not shown) provided in a housing of the carrier. An incoming air stream 42 is directed to the activation surface 38 of the fluid-transfer article 34 (e.g. via a fluid communication pathway within the housing of the carrier). When the incoming air stream 42 reaches a first side of the activation surface 38, the incoming air stream 42 flows across the activation surface 38 via the one or more channels 40. The air stream flowing through the one or more channels 40 is denoted by dashed line 44 in Figure 7. As the air stream 44 flows through the one or more channels 40, aerosol precursor at activation surface 38, across which the air stream 44 flows, is released from the activation surface 38 by heat conveyed to the activation surface from the heater elements 24. Aerosol precursor released from the activation surface 38 in this manner is then entrained in the air stream 44 flowing through the one or more channels 40.

In use, the heater elements 24 of the apparatus 12 convey heat to the fluid transfer article 34 to raise the temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article. As the air stream 44 continues its passage in the one or more channels 40, more released aerosol precursor is entrained within the air stream 44. When the air stream 44 entrained with aerosol precursor exits the one or more channels 40 at a second side of the activation surface 38, it is directed to an outlet, from where it can be inhaled by the user via a mouthpiece. An outgoing air stream 46 entrained with aerosol precursor is directed to the outlet (e.g. via a fluid communication pathway within the housing of the carrier).

Therefore, operation of the apparatus will cause heat from the heater elements 24 to be transferred to the activation surface 38 of the fluid-transfer article. At a sufficiently high temperature, captive substances held at the activation surface 38 of the fluid-transfer article 34 are released, or liberated, to form a vapour and/or aerosol. Thus, when a user draws on a mouthpiece of the apparatus, the released substances from the fluid-transfer article are drawn away from the activation surface 38 (entrained in a stream of air) and condense to form an aerosol that is drawn through the a gas communication pathway for delivery to an outlet, which is in fluid communication with the mouthpiece.

As the aerosol precursor is released from the activation surface 38, a wicking effect of the fluid-transfer article 34 causes aerosol precursor within the body of the fluid-transfer article to migrate to the activation surface 38 to replace the aerosol precursor released from the activation surface 38 into air stream 44.

Operation of the heater elements 24 is controlled by control circuitry (not shown), which is operable to actuate the heater elements 24 responsive to an actuation signal from a switch operable by a user or configured to detect when the user draws air through a mouthpiece of the apparatus by sucking or inhaling. In an optional arrangement, the control circuitry operates to actuate the heater elements 24 with as little delay as possible from receipt of the actuation signal from the switch, or detection of the user drawing air through the mouthpiece. This may effect near instantaneous heating of the activation surface 38 of the fluid-transfer article 34.

In the illustrated example of use of the apparatus schematically illustrated in Figure 8, rather than the case of Figure 7 where air is drawn toward the activation surface 38 from one side only (and exits from the one or more channels 40 at an opposite side), a gas communication pathway for an incoming air stream is configured to deliver the incoming air stream to the activation surface 38 from both sides of the fluid-transfer article, and thus from both ends of the channels 40 formed therein. In such an arrangement, a gas communication pathway for an outlet airstream may be provided through the body of the fluid-transfer article 34. An outlet fluid communication pathway for an outlet airstream in the illustrative example of Figure 8 is denoted by reference number 48.

Thus, in the illustrative example of Figure 8, when a user draws on a mouthpiece of the apparatus, air is drawn into the carrier 14 through inlet apertures (not shown) provided in a housing of the carrier. An incoming air stream 42a from a first side is directed to a first side of the activation surface 38 of the fluid-transfer article 34 (e.g. via a gas communication pathway within the housing of the carrier 14). An incoming air stream 42b from a second side is directed to a second side of the activation surface 38 of the fluid-transfer article 34 (e.g. via a gas communication pathway within the housing of the carrier 14). When the incoming air stream 42a from the first side reaches the first side of the activation surface 38, the incoming air stream 42a flows across the activation surface 38 via the one or more channels 40. Likewise, when the incoming air stream 42b from the second side reaches the second side of the activation surface 38, the incoming air stream 42b flows across the activation surface 38 via the one or more channels 40. The air streams 42a, 42b from each side flowing through the one or more channels 40 are denoted by dashed lines 44a and 44b in Figure 8. As air streams 44a and 44b flow through the one or more channels 40, aerosol precursor in the activation surface 38, across which the air streams 44a and 44b flow, is released from the activation surface 38 by heat conveyed to the activation surface from the heater elements 24. Aerosol precursor released from the activation surface 38 is entrained in air streams 44a and 44b flowing through the one or more channels 40.

In use, the heater elements 24 of the apparatus 12 convey heat to the fluid-transfer article 34 to raise a temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article. As the air streams 44a and 44b continue their passages in the one or more channels 40, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they exit outlet fluid communication pathway 48, either as a single outgoing air stream 46 (as shown), or as separate outgoing air streams. The outgoing air stream 46 is directed to an outlet, from where it can be inhaled by the user via a mouthpiece. The outgoing air stream 46 entrained with aerosol precursor is directed to the outlet (e.g. via a gas communication pathway within the housing of the carrier 14).

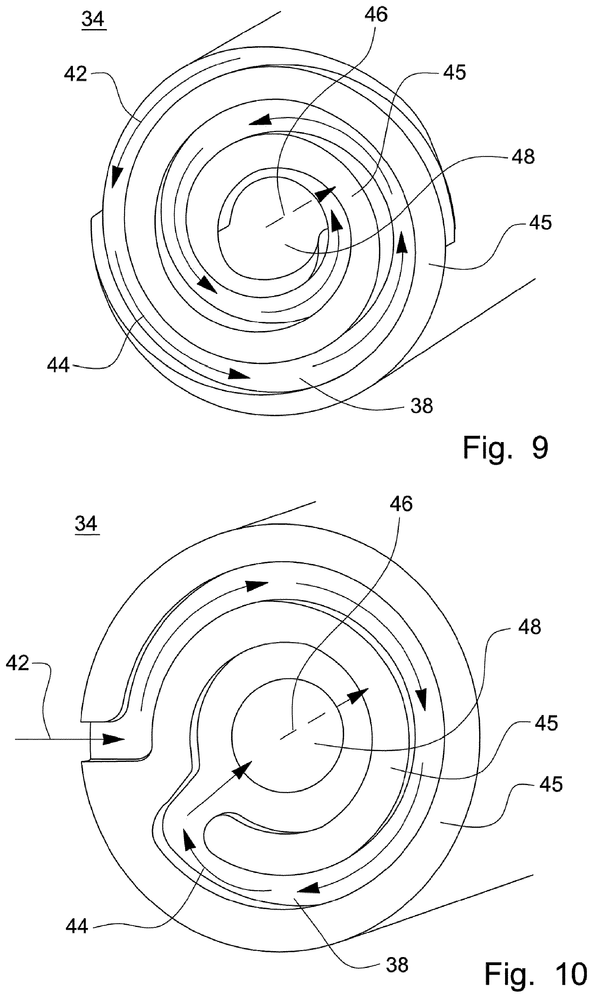

Figures 9 and 10 are perspective end view illustrations of a fluid-transfer article 34 of the aerosol carrier according to one or more arrangements. These figures show different types of channel configurations as illustrative examples. In both illustrative examples of a channel configuration, as shown in Figures 9 and 10, the fluid-transfer article 34 comprises a cylindrical member, which comprises a central bore extending therethrough for fluid communication between the activation surface 38 and an outlet, from where an outgoing air stream can be delivered for inhalation. The central bore serves as a fluid communication pathway 48 (e.g. as described above in relation to Figure 9). Note that, in the arrangements of Figures 9 and 10, the channels 40 extend radially and the sectional views of Figures 9 and 10 are along the length of two channels at opposite radial positions relative to the central bore of the fluid-transfer article. The heating elements 24 are therefore not visible in Figures 9 and 10, although they will be present in similar positions, relative to the channels 40, as the heating elements 24 and channels 40 in Figures 5 to 8.

In both illustrative examples of Figures 9 and 10, an incoming air stream 42 is directed to a mouth of a channel 40 formed between the activation surface 38 of the fluid-transfer article 34 and conduction element (not shown), or between the activation surface 38 and a heater (not shown). In both illustrative examples of Figures 9 and 10, the mouth of the channel 40 is located at an outer edge of the fluid-transfer article 34 and an exit from the channel 40 (in fluid communication with the fluid communication pathway 48) is located toward a centre of the fluid-transfer article. Therefore, the incoming air stream 42 enters the channel 40 via channel mouth at the outer edge of the fluid-transfer article 34 and moves toward the centre of the fluid-transfer article 34 as directed by the channel 40. As described above, as the air stream passes across activation surface 38 through channel 40, aerosol precursor is released from the activation surface 38 and is entrained in air stream 44. Air stream 44 continues to flow through the channel 40 until it reaches an exit thereof, from where it enters the fluid communication pathway 48 and proceeds as an outgoing air stream 46 entrained with aerosol precursor toward the outlet.

In both illustrative examples of Figures 9 and 10, the heater elements 24 are not shown, but they will be formed on the raised parts 45 of the activation surface, and optionally on part of the sides of the channels 40.

In both illustrative examples of Figures 9 and 10, the valleys or grooves of the activation surface 38 that form part of the channel 40 are arranged to define a circuitous route 20 across the activation surface. In the illustrative examples, the route is a spiral path, but in optional arrangements, may be meandering or circuitous in some other manner. In optional arrangements, the activation surface may be located to face outwardly from the cylinder, such that the groove(s) or valley(s) may be in the outer surface of the cylinder forming the fluid-transfer article. These grooves or valleys may be arranged in parallel in a direction along the length of the cylinder. The groove(s) or valley(s) may be arranged in a spiral manner around the outside of the cylinder. In optional arrangements, the activation surface 38 may be located to face inwardly from the cylinder (i.e. surrounding the central bore), such that the groove(s) or valley(s) may be in the inner surface of the cylinder forming the fluid-transfer article 34. These grooves or valleys may be arranged in parallel in a direction along the length of the cylinder. The groove(s) or valley(s) may be arranged in a spiral manner around the inside of the cylinder.

Figures 11 and 12 illustrate an aerosol carrier 14 according to one or more possible arrangements in more detail. Figure 11 is a cross-section side view illustration of the aerosol carrier 14 and Figure 12 is a perspective cross-section side view illustration of the aerosol carrier 14 of Figure 11.

As can be seen from Figures 11 and 12, the aerosol carrier 14 is generally tubular in form. The aerosol carrier 14 comprises housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 11 and 12, the fluid-transfer article 34 is an annular shaped element located around the fluid communication pathway 48, and the channels 40 formed so as to extend radially across its activation surface.

In walls of the housing 32, there are provided inlet apertures 50 to provide a fluid communication pathway for an incoming air stream to reach the fluid-transfer article 34, and particularly the one or more channels 40 in the activation surface of the fluid-transfer article 34. Note that, although the heater elements 24 are not shown in Figures 11 and 12, will be present on the activation surface 38 parts other than at the channels 40.

In the illustrated example of Figures 11 and 12, the aerosol carrier 14 further comprises a filter element 52. The filter element 52 is located across the fluid communication pathway 48 such that an outgoing air stream passing through the fluid communication pathway 48 passes through the filter element 52.

With reference to Figure 12, when a user sucks on a mouthpiece of the apparatus (or on the second end 18 of the aerosol carrier 14, if configured as a mouthpiece), air is drawn into the carrier through inlet apertures 50 extending through walls in the housing 32 of the aerosol carrier 14. An incoming air stream 42a from a first side of the aerosol carrier 14 is directed to a first side of the activation surface 38 of the fluid-transfer article 34 (e.g. via a gas communication pathway within the housing of the carrier). An incoming air stream 42b from a second side of the aerosol carrier 14 is directed to a second side of the activation surface 38 of the fluid-transfer article 34 (e.g. via a gas communication pathway within the housing of the carrier). When the incoming air stream 42a from the first side of the aerosol carrier 14 reaches the first side of the activation surface 38, the incoming air stream 42a from the first side of the aerosol carrier 14 flows across the activation surface 38 via the one or more channels 40 in the activation surface 38. Likewise, when the incoming air stream 42b from the second side of the aerosol carrier 14 reaches the second side of the activation surface 38, the incoming air stream 42b from the second side of the aerosol carrier 14 flows across the activation surface 38 via the one or more channels 40 in the activation surface 38. The air streams from each side flowing through the one or more channels 40 are denoted by dashed lines 44a and 44b in Figure 12. As air streams 44a and 44b flow through the one or more channels 40, aerosol precursor in the activation surface 38, across which the air streams 44a and 44b flow, is released from the activation surface 38 by heat conveyed to the activation surface from the heater elements 24. Aerosol precursor released from the activation surface 38 is entrained in air streams 44a and 44b flowing through the one or more channels 40.

In use, the heater elements 24 of the apparatus 12 convey heat to the activation surface 38 of the fluid-transfer article 34 to raise a temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article 34. As the air streams 44a and 44b continue their passages in the one or more channels 40, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

When the user initially draws on a mouthpiece of the apparatus (or one the second end 18 of the aerosol carrier 14, if configured as a mouthpiece), this will cause an air column located in the fluid communication pathway 48 to move towards the outlet. In tum, this will draw air into the fluid communication pathway from the one or more channels 40. This will cause a pressure drop in the channels 40. To equalise the pressure in the channels 40, air will be drawn into the aerosol carrier 14, and thus into the channels 40 via the inlet apertures 50. During the period of lower pressure in the one or more channels 40 when the user begins to draw, aerosol precursor in the fluid-transfer medium will be released into the channels from the activation surface 38, because the aerosol precursor is drawn into the one or more channels by way of the lower pressure. This effect is in addition to the effect of releasing the aerosol precursor from the activation surface 38 by way of heat conveyed from the heater. The drawing of the aerosol precursor from the activation surface 38 by way of the user sucking on the mouthpiece of the apparatus (or one the second end 18 of the aerosol carrier 14, if configured as a mouthpiece) may produce a dragging effect on the volumetric rate of flow experienced by the user during a suction action, i.e. the user may have to suck harder to achieve a same volumetric rate of flow. This effect may manifest itself as a similar physical sensation experienced by the user as those experienced from a traditional smoking or tobacco product.

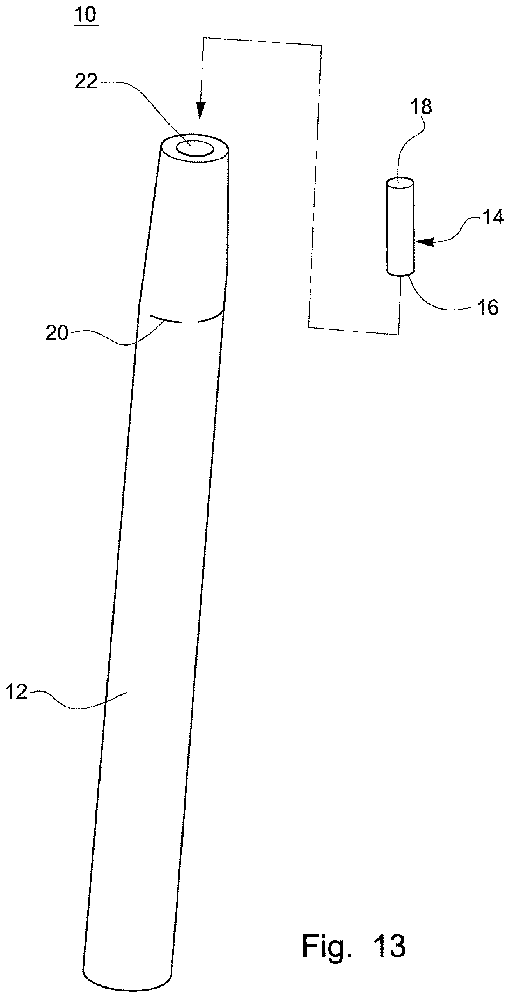

Figure 13 is an exploded perspective view illustration of a kit-of-parts for assembling an aerosol delivery system 10.

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article 34, whilst also allowing the carrier 14 to be handled by a user without his/her fingers coming into contact with the aerosol precursor liquid retained therein. In such arrangements, it will be appreciated that the carrier 14 has a multi-part construction. In some cases this might be considered somewhat disadvantageous because it requires a relatively complicated assembly procedure which can be both time-consuming and expensive.

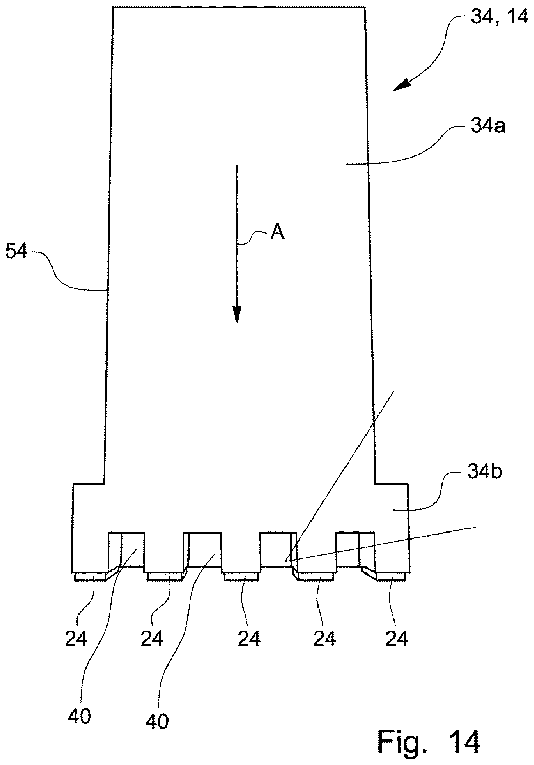

Turning now to consider Figure 14, there is illustrated another possible aspect of the fluid-transfer article 34, which may be employed in some arrangements, and which may permit the creation of a significantly simplified carrier 14.

Figure 14 illustrates an alternative fluid-transfer article 34 with air flow channels 40 in the activation surface 38. In the arrangement of Figure 14, the substrate forming the fluid-transfer article 34 again comprises a porous material where pores of the porous material hold, contain, carry, or bear the aerosol precursor material. It is envisaged, for example, that the same types of substrate material may be used in the arrangement illustrated in Figure 14 as in the previously-described arrangements. In particular, therefore, the porous material of the fluid-transfer article 34 may be a polymeric wicking material. However, in the arrangement illustrated in Figure 14, the substrate material includes an integrally formed peripheral wall 54.

It is proposed that the peripheral wall 54 may be formed by treating the outermost surface of the porous substrate material of the fluid-transfer article 34 so as to render the surface substantially liquid-impermeable. For example, it is envisaged that in some arrangements the substrate material may be locally heated so as to fuse the material and close up its internal pores in the localised region of the surface. Alternatively, it is envisaged that the substrate material may be treated by a sintering process in order to create the liquid-impermeable peripheral wall 54. The peripheral wall 54 may alternatively be created by a chemical treatment process to render the substrate material substantially liquid-impermeable in the region of its outermost surface. As will therefore be appreciated, the peripheral wall 54 may be considered to take the form of a skin formed from the material of the substrate itself.

The peripheral wall may be created in this manner so as to substantially completely circumscribe the substrate material. It is to be appreciated, however, that the activation surface 38 of the fluid-transfer article 34 will not be treated in this manner, thereby ensuring that it will retain the function described above in detail in cooperation with the heater elements 24. The thickness of the peripheral wall 54 formed from the substrate may vary depending on the desired physical properties of the fluid-transfer article 34. For example, a relatively thin wall 54 might be desirable in some circumstances, as this may retain some flexibility in the material, thereby providing a fluid-transfer article which will feel soft in the hands of a user. Alternatively, a relatively thick peripheral wall 54 might be desirable in arrangements where the wall 54 is required to provide some structural rigidity to the fluid-transfer article 34. The wall 54 may therefore have a thickness of less than 3mm; or less than 2.5mm; or less than 2mm; or less than 1.5mm; or less than 1 mm; or less than 0.9mm; or less than 0.8mm; or less than 0.7mm; or less than 0.6mm; or less than 0.5mm; or less than 0.4mm; or less than 0.3mm; or less than 0.2mm; or less than 0.1mm in some embodiments.

As will be appreciated, the liquid-impermeable nature of the resulting peripheral wall or skin means that the fluid-transfer article 34 may be handled by a user without getting his or her fingers wet from the aerosol precursor liquid retained therein. This opens up the possibility of the fluid-transfer article 34 being used without an enclosing housing 32, as was necessary in the previously-described arrangements. It is therefore envisaged that in some arrangements, the fluid-transfer article 34 may itself define an entire aerosol carrier 14. Furthermore, it is envisaged that in some embodiments, a fluid-transfer article 34 in accordance with this proposal may be provided in the form of a unitary monolithic element of substrate material and could, therefore, take the form of a single-piece consumable or carrier 14 for an aerosol-delivery system 10, which may be provided pre-filled with aerosol precursor liquid and which may be discarded when the initial volume of precursor has been used. A single-piece consumable of this type offers very significant advantages in terms of cost of manufacture, and from an environmental point of view.

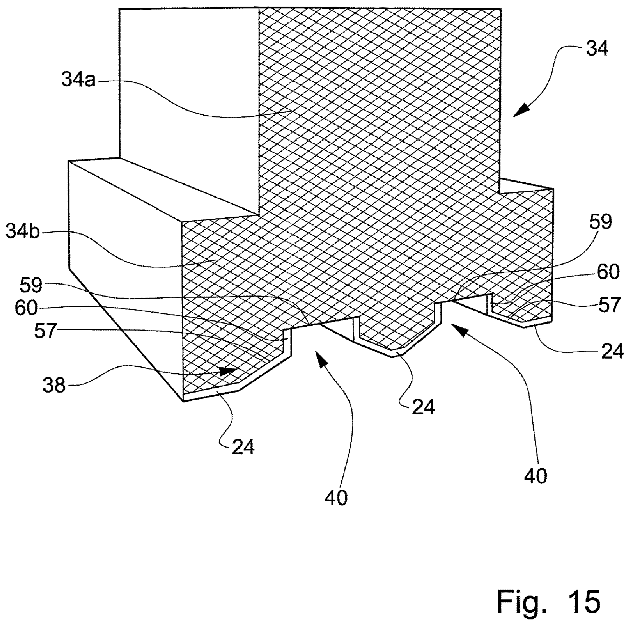

Turning now to consider Figure 15, there is illustrated a fluid-transfer article 34 in combination with heater elements 24. The fluid-transfer article 34 may have a peripheral wall or skin formed in the manner described above, although this is not essential and indeed is not present in the particular arrangement illustrated in figure 15. The particular feature of the fluid-transfer article 34 illustrated in figure 15 which is of relevance is the cross-sectional profile of the channels 40 defined in the activation surface 38 of the second region 34b of the article. As will be noted, the channels 40 visible in figure 15 have a significantly different profile to the "saw-tooth" type profile illustrated in Figure 5, and to the "castellated" type profile illustrated in Figure 6. Nevertheless, as will be explained, the channel profile illustrated in Figure 15 shares a characteristic with the "saw-tooth" type profile illustrated in Figure 5.

The activation surface 38 of the arrangement of Figure 15 is discontinuous in a manner such that it includes a plurality of spaced-apart angled surface portions 57. The angled surface portions 57 of the activation surface 38 are arranged in pairs, the members of each pair cooperating to define opposing angled walls of a respective channel 40. In the particular channel configuration illustrated in Figure 15, each channel 40 also comprises a respective ceiling portion 59 between the two spaced-apart angled surfaces 57. Each channel 40 further comprises a pair of opposed side walls 60, each of which interconnects an edge of a respective angled surface portion 57 and a respective side edge of the ceiling portion 59.

In the arrangement of Figure 15, the heater elements 24 cover not only the peaks of the activation surface 38, but also the angled surface portions 57 in the side walls 60. This maximises the heat that can be transferred to the fluid-transfer article 34, but it also has the effect of limiting the parts of the activation surface from which aerosol precursor can pass to the ceiling portions 59. If this is found to be insufficient, the side walls 57 may be left uncovered by the heater elements 24.

In the arrangement illustrated in Figure 15, the ceiling portion 59 of each channel 40 presents a substantially planar surface in spaced-relation to the heating surface 55. However, as will be explained below in relation to Figure 6, in other arrangements the ceiling portion may alternatively present an arcuate surface towards the heating surface 55.

As will be observed, the "saw-tooth" channel profile illustrated in Figure 5 can be considered somewhat similar to the profile illustrated in Figure 15, in the sense that it is also defined by an activation surface 38 which is discontinuous in a manner effective to include angled surface portions arranged to form acute intersection angles with a planar heating surface.

It is believed that the aforementioned angled surfaces 57, and more particularly their acute intersection angles 58 to the heater surface 55 aid in the release of liquid aerosol precursor from the substrate material of the fluid-transfer article 34. It is believed that the sharp corners defined at the angled points of intersection between the angled surfaces 57 and the heater surface 55 create improved vaporisation sites for the release of aerosol precursor, and allow the liquid to form menisci along the corner edges of the channels 40, at the sites of the acute angles 58, on the heating surface 55. This has been found to promote more efficient and quicker heating and vaporisation of the precursor liquid at the heating surface 55.

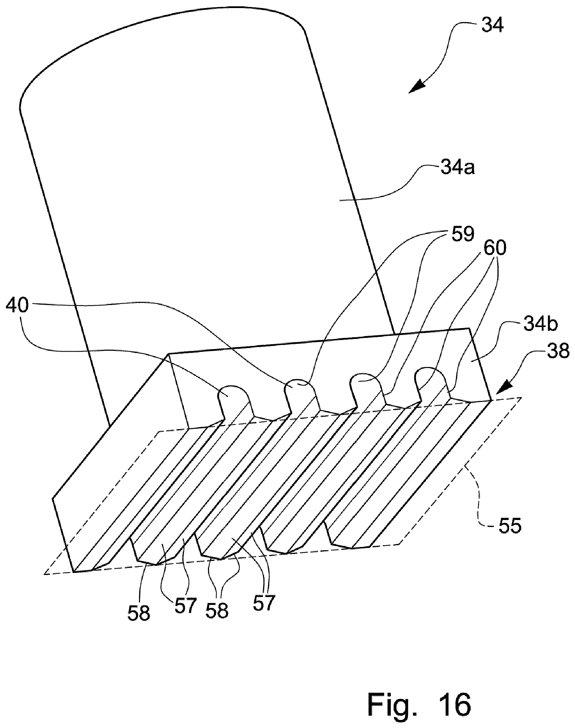

Turning now to consider Figure 16, there is illustrated a fluid-transfer article 34 having another configuration of activation surface 38. The fluid-transfer article 34 is illustrated as viewed from below the activation surface 38, and in the absence of heater elements 24 for the sake of clarity.

As will be observed from Figure 16, the activation surface 38 is again discontinuous in a manner such that it includes a plurality of spaced-apart angled surface portions 57, each of which will form an acute intersection angle with a plane 55 aligned with their peaks. In the particular arrangement illustrated, the intersection angles are considerably smaller than in the configuration illustrated in Figure 15 and may, for example, be less than 20 degrees, although this is not essential.

Furthermore, it will also be observed that the angled surface portions 57 of the activation surface 38 are again arranged in pairs, the members of each pair cooperating to define opposing angled walls of a respective channel 40. In the particular channel configuration illustrated in Figure 16, each channel 40 also comprises a respective pair of generally planar side walls 60 which are oriented so as to be substantially perpendicular to the plane 55 when the fluid-transfer article 34 and a heater 24 (or conduction element 36) are interengaged. The side walls 60 extend upwardly from the edges of respective angled surface portions 57, and are interconnected by a ceiling portion 59 of the respective channel 40. In this arrangement, the ceiling portion 59 of each channel 40 defines an arcuate surface portion, which it will be understood forms part of the discontinuous activation surface 38 of the fluid-transfer article. The arcuate surface portion of each channel 40 is arranged to oppose the heating surface 55, in spaced-relation thereto, and is concave towards the plane 55.

In such an arrangement, the heater elements 24 may be formed on the angled surface portions 57, and possibly also on parts of the side walls 60.

In preferred arrangements of the type illustrated in Figure 16, it is proposed that the arcuate ceiling portion 59 of each channel will blend smoothly into the side walls 60 of the channel, thereby eliminating sharp corner edges in the upper region of the channel 40. Such sharp corners can be seen, by comparison, in the arrangement of Figure 15, and are denoted at 61. It has been found that by eliminating such sharp corners from the upper regions of the channels' cross-sectional profile, more efficient release or liberation of the aerosol precursor liquid from the porous substrate of the fluid-transfer article 34 may be achieved. This is because it has been found that liquid held in the wicking material has a tendency to collect at sharp corners of the channel profiles. This can present a disadvantage at the top of each channel 40. This is because if excessive precursor liquid collects around the upper regions of the channel 40, it can be drawn out of the porous wicking material and sufficiently away from the heating surface 55 by the airflow through the channel without having been heated and thus vaporised by contact with the heating surface.

In some arrangements, the or each channel 40 may be configured to have the above-described arrangement of spaced-apart side walls 60 and interconnecting arcuate ceiling portion 59 without the provision of the above-described angled surface portions 57, such that the lower edges of the side walls 60 will be configured for direct contact with the heating surface 55 of a heater 24 or conduction element 36.

The porous layer may have a thickness of less than 5mm. In other embodiments it may have a thickness of: less than 3.5mm, less than 3mm, less than 2.5mm, less than 2mm, less than 1.9mm, less than 1.8mm, less than 1.7mm, less than 1.6mm, less than 1.5mm, less than 1.4mm, less than 1.3mm, less than 1.2mm, less than 1.1mm, less than 1mm, less than 0.9mm, less than 0.8mm, less than 0.7mm, less than 0.6mm, less than 0.5mm, less than 0.4mm, less than 0.3mm, less than 0.2mm, or less than 0.1mm.

There has been described in the foregoing one or more proposals for an aerosol delivery system, and parts thereof, that avoids or at least ameliorates problems of the prior art.

In one or more optional arrangements, a fluid-transfer article 34 containing nicotine and/or nicotine compounds may be substituted or supplemented with a fluid-transfer article configured to provide a flavoured vapour and/or aerosol upon heating of the fluid-transfer article by the heater elements 24 of the apparatus 12. A precursor material for forming the flavoured vapour and/or aerosol upon heating is held within pores, spaces, channels and/or conduits within the fluid-transfer article. The precursor material may be extracted from a tobacco plant starting material using a supercritical fluid extraction process. Optionally, the precursor material is nicotine-free and comprises tobacco-flavours extracted from the tobacco plant starting material. Further optionally, the extracted nicotine-free precursor material (e.g. flavours only) could have nicotine added thereto prior to loading of the precursor material into the substrate of the carrier unit. Further optionally, flavours and physiologically active material may be extracted from plants other than tobacco plants.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. An aerosol-generation apparatus (12) comprising a heater (24) and a fluid-transfer article (34), the fluid-transfer article (34) having a first region (34a) for holding an aerosol precursor and for transferring said aerosol precursor to an activation surface (38) of a second region (34b) of said article (34), wherein said second region (34b) comprises at least one discontinuity in said activation surface (38) to form a corresponding at least one channel (40) in said activation surface (38), the or each said channel (40) being configured for providing an air-flow pathway across said activation surface (38) and opening in a direction away from said first region (34a), **characterised by** said heater (24) having at least one heater element (24) formed on said activation surface (38), and said at least one heater element (24) being of a dried conductive fluid with electrical connections thereto.

2. An aerosol-generation apparatus (12) according to claim 1, wherein said at least one heater element (24) is formed on parts of said activation surface (38) other than at the or each discontinuity.

3. An aerosol-generation apparatus (12) according to claim 2, wherein at least parts of said at least one heating element (24) are formed on parts of said activation surface (38) between said channels (40).

4. An aerosol-generation apparatus (12) according to any one of the preceding claims, wherein the or each said channel (40) is at least partly defined by a pair of spaced apart side walls (60) and an arcuate surface portion extending between said wall portions (60) to form a ceiling portion (59) of said channel (40).

5. An aerosol-generation apparatus (12) according to claim 4, wherein said arcuate surface portion blends smoothly with each of said side walls (60), thereby eliminating a sharp corner therebetween.

6. An aerosol-generation apparatus (12) according to any one of claims 1 to 3, wherein the or each channel (40) is a least partially defined by a pair of spaced apart side walls (60) and a flat surface portion, said flat surface portion extending between said wall portions (60) to form a ceiling portion (59) of said channel (40).

7. An aerosol-generation apparatus (12) according to any one of claims 1 to 3, wherein the or each channel (40) is at least partially defined by a pair of side walls (60), said side walls (60) being inclined relative to each other to meet at an apex portion of said channel (40).

8. An aerosol-generation apparatus (12) according to any one of claims 4 or claim 7, wherein said side walls (60) are substantially planar.

9. An aerosol-generation apparatus (12) according to any of claims 4 to 8, wherein said at least one heater element (24) at least partially covers said side walls (60).

10. An aerosol-generation apparatus (12) according to any preceding claim, wherein at least said second region (34b) is formed from a polymeric wicking material.

11. An aerosol-generation apparatus (12) according to claim 10, wherein said first and second regions (34a, 34b) are both formed from said polymeric wicking material.

12. An aerosol-generation apparatus (12) according to claim 10 or claim 11, wherein said polymeric wicking material is porous.

13. An aerosol-generation apparatus (12) according to any one of claims 10 to 12, wherein said polymeric wicking material is configured such that pore diameter in said first region (34a) is greater than pore diameter in said second region (34b).

14. An aerosol-generation apparatus (12) according to any one of claims 10 to 13, wherein said polymeric wicking material is heat resistant.

15. An aerosol-generation apparatus (12) according to any one of claims 10 to 14, wherein said polymeric wicking material is a hydrophilic material that is configured to transfer fluid from said first region (34a) to said second region (34b).

16. An aerosol-generation apparatus (12) according to any one of claims 10 to 15, wherein said polymeric wicking material is of greater hydrophilicity in said second region (34b) than said first region (34a).

17. An aerosol-delivery system (10) comprising: an aerosol-generation apparatus (12) according to any one of the preceding claims and a carrier (14), the carrier (14) having a housing (32) containing the heater (24) and the fluid-transfer article (34).

18. An aerosol-delivery system (10) according to claim 17, wherein said housing (32) has an inlet (50) and an outlet and said air-flow pathway (48) extends to said inlet (50) and said outlet.

19. A method of forming an aerosol-generation device comprising:
forming a fluid-transfer article (34), the fluid-transfer article (34) having a first region (34a) for holding an aerosol precursor and for transferring said aerosol precursor to an activation surface (38) of a second region (34b) of said article (34), wherein said second region (34b) comprises at least one discontinuity in said activation surface (38) to form a corresponding at least one channel (40) in said activation surface (38), the or each said channel (40) being configured for providing an air-flow pathway across said activation surface (38) and opening in a direction away from said first region (34a); and **characterised by**:
dipping said activation surface (38) in a conductive fluid to coat at least a part of said activation surface (38) with said conductive fluid;
drying said conductive fluid to form heater elements (24); and
making electrical connection to said dried conductive fluid, thereby to form a heater (24) on said fluid-transfer article (34).

## Patentansprüche

1. Aerosol-Erzeugungsvorrichtung (12), die eine Heizvorrichtung (24) und einen Fluidtransportartikel (34) umfasst, wobei der Fluidtransportartikel (34) eine erste Region (34a) zum Speichern eines Aerosol-Vorläufers und zum Transport des Aerosol-Vorläufers zu einer Aktivierungsoberfläche (38) einer zweiten Region (34b) des Artikels (34) aufweist, wobei die zweite Region (34b) zumindest eine Diskontinuität in der Aktivierungsoberfläche (38) aufweist, um zumindest einen entsprechenden Kanal (40) in der Aktivierungsoberfläche (38) zu bilden, wobei der oder jeder Kanal (40) ausgelegt ist, einen Luftströmungsweg über die Aktivierungsoberfläche (38) bereitzustellen und sich in eine Richtung weg von der ersten Region (34a) zu öffnen, **dadurch gekennzeichnet, dass** die Heizvorrichtung (24) zumindest ein Heizelement (24) aufweist, das auf der Aktivierungsoberfläche (38) ausgebildet ist, und das zumindest eine Heizelement (24) aus einem getrockneten leitenden Fluid mit elektrischen Verbindungen zu diesem besteht.

2. Aerosol-Erzeugungsvorrichtung (12) nach Anspruch 1, wobei das zumindest eine Heizelement (24) auf anderen Teilen der Aktivierungsoberfläche (38) als an der oder jeder Diskontinuität ausgebildet ist.

3. Aerosol-Erzeugungsvorrichtung (12) nach Anspruch 1, wobei zumindest Teile des zumindest einen Heizelements (24) auf Teilen der Aktivierungsoberfläche (38) zwischen den Kanälen (40) ausgebildet sind.

4. Aerosol-Erzeugungsvorrichtung (12) nach einem der vorangegangenen Ansprüche, wobei der oder jeder Kanal (40) zumindest teilweise durch ein Paar beabstandeter Seitenwände (60) und einen gebogenen Oberflächenabschnitt, der sich zwischen den Wandabschnitten (60) erstreckt, um einen Deckenabschnitt (59) des Kanals (40) zu bilden, definiert ist.

5. Aerosol-Erzeugungsvorrichtung (12) nach Anspruch 4, wobei der gebogene Oberflächenabschnitt nahtlos in jede der Seitenwände (60) übergeht, wodurch spitze Ecken dazwischen eliminiert werden.

6. Aerosol-Erzeugungsvorrichtung (12) nach einem der Ansprüche 1 bis 3, wobei der oder jeder Kanal (40) zumindest teilweise durch ein Paar beabstandeter Seitenwände (60) und einen flachen Oberflächenabschnitt definiert ist, wobei sich der flache Oberflächenabschnitt zwischen den Wandabschnitten (60) erstreckt, um einen Deckenabschnitt (59) des Kanals (40) zu bilden.

7. Aerosol-Erzeugungsvorrichtung (12) nach einem der Ansprüche 1 bis 3, wobei der oder jeder Kanal (40) zumindest teilweise durch ein Paar beabstandeter Seitenwände (60) definiert ist, wobei die Seitenwände (60) in Bezug zueinander geneigt sind und am Spitzenabschnitt des Kanals (40) zusammentreffen.

8. Aerosol-Erzeugungsvorrichtung (12) nach einem der Ansprüche 4 bis 7, wobei die Seitenwände (60) im Wesentlichen planar sind.

9. Aerosol-Erzeugungsvorrichtung (12) nach einem der Ansprüche 4 bis 8, wobei das zumindest eine Heizelement (24) die Seitenwände (60) zumindest teilweise bedeckt.

10. Aerosol-Erzeugungsvorrichtung (12) nach einem der vorangegangenen Ansprüche, wobei zumindest die zweite Region (34b) aus einem polymeren Dochtmaterial besteht.

11. Aerosol-Erzeugungsvorrichtung (12) nach Anspruch 10, wobei die erste und die zweite Region (34a, 34b) beide aus dem polymeren Dochtmaterial bestehen.

12. Aerosol-Erzeugungsvorrichtung (12) nach Anspruch 10 oder 11**,** wobei das polymere Dochtmaterial porös ist.

13. Aerosol-Erzeugungsvorrichtung (12) nach einem der Ansprüche 10 bis 12, wobei das polymere Dochtmaterial so ausgelegt ist, dass der Porendurchmesser in der ersten Region (34a) größer ist als der Porendurchmesser in der zweiten Region (34b).

14. Aerosol-Erzeugungsvorrichtung (12) nach einem der Ansprüche 10 bis 13, wobei das polymere Dochtmaterial hitzebeständig ist.

15. Aerosol-Erzeugungsvorrichtung (12) nach einem der Ansprüche 10 bis 14, wobei das polymere Dochtmaterial ein hydrophiles Material ist, das ausgelegt ist, Fluid von der ersten Region (34a) zur zweiten Region (34b) zu transportieren.

16. Aerosol-Erzeugungsvorrichtung (12) nach einem der Ansprüche 10 bis 15, wobei das polymere Dochtmaterial in der Region (34b) eine höhere Hydrophilie aufweist als in der ersten Region (34a).

17. Aerosol-Abgabesystem (10), das Folgendes umfasst: eine Aerosol-Erzeugungsvorrichtung (12) nach einem der vorangegangenen Ansprüche und einen Träger (14), wobei der Träger (14) ein Gehäuse (32) aufweist, das die Heizvorrichtung (24) und den Fluidtransportartikel (34) enthält.

18. Aerosol-Abgabesystem (10) nach Anspruch 17, wobei das Gehäuse (32) einen Einlass (50) und einen Auslass aufweist und sich der Luftströmungsweg (48) zu dem Einlass (50) und dem Auslass erstreckt.

19. Verfahren zur Ausbildung einer Aerosol-Erzeugungsvorrichtung, das Folgendes umfasst:
Ausbilden eines Fluidtransportartikels (34), wobei der Fluidtransportartikel (34) eine erste Region (34a) zum Speichern eines Aerosol-Vorläufers und zum Transport des Aerosol-Vorläufers zu einer Aktivierungsoberfläche (38) einer zweiten Region (34b) des Artikels (34) aufweist, wobei die zweite Region (34b) zumindest eine Diskontinuität in der Aktivierungsoberfläche (38) umfasst, um zumindest einen entsprechenden Kanal (40) in der Aktivierungsoberfläche (38) auszubilden, wobei der oder jeder Kanal (40) ausgelegt ist, einen Luftströmungsweg über die Aktivierungsoberfläche (38) bereitzustellen und sich in eine Richtung weg von der ersten Region (34a) zu öffnen; und **gekennzeichnet durch**:
Eintauchen der Aktivierungsoberfläche (38) in ein leitendes Fluid, um zumindest einen Teil der Aktivierungsoberfläche (38) mit dem leitenden Fluid zu beschichten;
Trocknen des leitenden Fluids, um Heizelemente (24) auszubilden; und
Herstellen einer elektrischen Verbindung zu dem getrockneten leitenden Fluid, um so eine Heizvorrichtung (24) auf dem Fluidtransportartikel (34) auszubilden.

## Revendications

1. Appareil de génération d'aérosol (12) comprenant un dispositif de chauffage (24) et un article de transfert de fluide (34), l'article de transfert de fluide (34) présentant une première région (34a) destinée à contenir un précurseur d'aérosol et à transférer ledit précurseur d'aérosol vers une surface d'activation (38) d'une seconde région (34b) dudit article (34), dans lequel ladite seconde région (34b) comprend au moins une discontinuité dans ladite surface d'activation (38) pour former au moins un canal correspondant (40) dans ladite surface d'activation (38), le ou chaque dit canal (40) étant configuré pour fournir un trajet d'écoulement d'air à travers ladite surface d'activation (38) et s'ouvrant dans une direction s'éloignant de ladite première région (34a), **caractérisé en ce que** ledit dispositif de chauffage (24) présente au moins un élément chauffant (24) formé sur ladite surface d'activation (38), et ledit au moins un élément de chauffage (24) est un fluide conducteur séché avec des connexions électriques à celui-ci.

2. Appareil de génération d'aérosol (12) selon la revendication 1, dans lequel ledit au moins un élément chauffant (24) est formé sur des parties de ladite surface d'activation (38) autres qu'au niveau de la ou de chaque discontinuité.

3. Appareil de génération d'aérosol (12) selon la revendication 2, dans lequel au moins des parties dudit au moins un élément chauffant (24) sont formées sur des parties de ladite surface d'activation (38) entre lesdits canaux (40).

4. Appareil de génération d'aérosol (12) selon l'une quelconque des revendications précédentes, dans lequel le ou chaque dit canal (40) est au moins partiellement défini par une paire de parois latérales espacées (60) et une partie de surface arquée s'étendant entre lesdites parties de paroi (60) pour former une partie de plafond (59) dudit canal (40).

5. Appareil de génération d'aérosol (12) selon la revendication 4, dans lequel ladite partie de surface arquée se fond en douceur avec chacune desdites parois latérales (60), en éliminant ainsi un coin aigu entre celles-ci.

6. Appareil de génération d'aérosol (12) selon l'une quelconque des revendications 1 à 3, dans lequel le ou chaque canal (40) est au moins partiellement défini par une paire de parois latérales espacées (60) et une partie de surface plate, ladite partie de surface plate s'étendant entre lesdites parties de paroi (60) pour former une partie de plafond (59) dudit canal (40).

7. Appareil de génération d'aérosol (12) selon l'une quelconque des revendications 1 à 3, dans lequel le ou chaque canal (40) est au moins partiellement défini par une paire de parois latérales (60), lesdites parois latérales (60) étant inclinées l'une par rapport à l'autre pour se rencontrer au niveau d'une partie de sommet dudit canal (40).

8. Appareil de génération d'aérosol (12) selon la quelconque des revendications 4 ou 7, dans lequel lesdites parois latérales (60) sont sensiblement planes.

9. Appareil de génération d'aérosol (12) selon l'une quelconque des revendications 4 à 8, dans lequel ledit au moins un élément chauffant (24) recouvre au moins partiellement lesdites parois latérales (60).

10. Appareil de génération d'aérosol (12) selon l'une quelconque des revendications précédentes, dans lequel au moins ladite seconde région (34b) est formée à partir d'un matériau polymère à effet de mèche.

11. Appareil de génération d'aérosol (12) selon la revendication 10, dans lequel lesdites première et seconde régions (34a, 34b) sont toutes deux formées à partir dudit matériau polymère à effet de mèche.

12. Appareil de génération d'aérosol (12) selon la revendication 10 ou la revendication 11, dans lequel ledit matériau polymère à effet de mèche est poreux.

13. Appareil de génération d'aérosol (12) selon la quelconque des revendications 10 à 12, dans lequel ledit matériau polymère à effet de mèche est configuré de telle sorte qu'un diamètre de pore dans ladite première région (34a) est supérieur à un diamètre de pore dans ladite seconde région (34b).

14. Appareil de génération d'aérosol (12) selon l'une quelconque des revendications 10 à 13, dans lequel ledit matériau polymère à effet de mèche est thermorésistant.

15. Appareil de génération d'aérosol (12) selon l'une quelconque des revendications 10 à 14, dans lequel ledit matériau polymère à effet de mèche est un matériau hydrophile qui est configuré pour transférer un fluide de ladite première région (34a) à ladite seconde région (34b).

16. Appareil de génération d'aérosol (12) selon l'une quelconque des revendications 10 à 15, dans lequel ledit matériau polymère à effet de mèche est plus hydrophile dans ladite seconde région (34b) que dans ladite première région (34a).

17. Système de distribution d'aérosol (10) comprenant :un appareil de génération d'aérosol (12) selon l'une quelconque des revendications précédentes et un support (14), le support (14) présentant un logement (32) contenant le dispositif de chauffage (24) et l'article de transfert de fluide.

18. Système de distribution d'aérosol (10) selon la revendication 17, dans lequel ledit logement (32) présente une entrée (50) et une sortie et ledit trajet d'écoulement d'air (48) s'étend jusqu'à ladite entrée (50) et ladite sortie.

19. Procédé de formation d'un dispositif de génération d'aérosol comprenant l'étape consistant à :
former un article de transfert de fluide (34), l'article de transfert de fluide (34) présentant une première région (34a) destinée à contenir un précurseur d'aérosol et à transférer ledit précurseur d'aérosol vers une surface d'activation (38) d'une seconde région (34b) dudit article (34), dans lequel ladite seconde région (34b) comprend au moins une discontinuité dans ladite surface d'activation (38) pour former au moins un canal correspondant (40) dans ladite surface d'activation (38), le ou chaque dit canal (40) étant configuré pour fournir un trajet d'écoulement d'air à travers ladite surface d'activation (38) et s'ouvrant dans une direction s'éloignant de ladite première région (34a) ; et **caractérisé par** les étapes consistant à :
immerger ladite surface d'activation (38) dans un fluide conducteur pour revêtir au moins une partie de ladite surface d'activation (38) avec ledit fluide conducteur ;
sécher ledit fluide conducteur pour former des éléments chauffants (24) ; et
établir une connexion électrique avec ledit fluide conducteur séché, pour former ainsi un dispositif de chauffage (24) sur ledit article de transfert de fluide (34).
